# EUROPEAN PATENT APPLICATION

(11) **EP 2 612 594 A2**
(43) Date of publication of application: **10.07.2013**
(21) Application number: 13150414.4
(22) Date of filing: 07.01.2013
(51) Int. Cl.: A61B 5/024, A61B 5/00

(54) **Heart rate monitor**

(30) Priority: 05.01.2012 US 201261583532 P
(71) Applicant: SCOSCHE INDUSTRIES, INC., Oxnard, CA 93033 (US)
(72) Inventor: Buchheim, James, Oxnard, CA California 93033 (US); Hennig, Arne, Fort Lauderdale, FL Florida 33328 (US)
(74) Representative: Kensett, John Hinton

(57) **Abstract**

A heart rate monitor is disclosed comprising two main components: a user system in form of a wristwatch type device including a plurality of sensors, and a second device, such as a smart phone, that receives and processes the transmitted sensor signals. The user system includes an optical blood concentration sensing system (310) for detecting the heart rate, a motion sensor (320) to sense changes in the position of the user system with respect to the skin in order to compensate for noise, and an accelerometer (330) to provide information about the motion of the user system with respect to the user's heart. A heart rate Kalman filter is configured to compute a heart rate on the basis of signals obtained from the plurality of sensors.

## Description

### BACKGROUND

### Field

The present disclosure relates generally to health monitoring systems and methods, and more particularly to monitoring heart rate under various conditions of exercise.

### Background

A pulse is the rate at which the heart beats, measured in beats per minute (bpm). Basil pulse is the pulse measured at rest. The pulse measured during physical activity is generally higher than the basil pulse, and the rise in pulse during physical exertion is a measure of the efficiency of the heart in response to demand for blood supply.

A person engaging in physical activity often wishes to monitor the heart rate via pulse measurement in order to monitor and/or regulate the degree of exertion, depending on whether the exercise is intended for fitness maintenance, weight maintenance/reduction, cardiovascular training, or the like.

A standard method of measuring pulse manually is to apply gentle pressure to the skin where an artery comes close to the surface, e.g., at the wrist, neck, temple area, groin, behind the knee, or top of the foot. However, measuring pulse this way during exercise is usually not feasible. Therefore, numerous devices provide pulse measurement using any of a variety of sensors attached to the body in some fashion. Monitors attached to the wrist, chest, ankle and upper arm, are preferably placed over a near-skin artery, are common. The method of measurement may involve skin contact electrodes.

A wireless heart rate monitor conventionally consists of a chest strap sensor-transmitter and a wristwatch-type receiver. The chest strap sensor has to be worn around the chest during exercise. It has two electrodes, which are in constant contact with the skin, to detect electrical activities coming from the heart. Once the chest strap sensor-transmitter has picked up the heart signals, it transmits the information wirelessly and continuously to the wristwatch. The number of heart beats per minute is then calculated and the value displayed on the wristwatch.

The wireless heart rate monitor can be further subdivided into digital and analog, depending on the wireless technology the chest strap sensor-transmitter uses to transmit information to the wristwatch. The wireless heart rate monitor with analog chest strap sensor-transmitter is a popular type of heart rate monitors. There is, however, a possibility of signal interference (cross-talk) if other analogue heart rate monitor users are exercising nearby. If that happens, the wristwatch may not accurately display the wearer's heart rate.

One type of analog chest strap sensor-transmitter transmits coded analog wireless signals. Coded analog transmission tend to reduce (but not eliminate entirely) the degree of cross talk while simultaneously preserving the ability to interface with remote heart rate monitor equipment.

A digital chest strap sensor-transmitter eliminates the problem of cross-talk when other heart rate monitor users are close by. By its very nature, the digital chest strap sensor transmitter is engineered to talk only to its own receiver (e.g., wristwatch).

Strapless heart rate monitors are wristwatch-type devices that may be preferred by users engaged in physical training because of convenience and combined time keeping features. In some cases the user is required to press a conductive contact on the face of the device to activate a pulse measurement sequence based on electrical sensing at the finger tip. However, this may require the user to interrupt physical activity, and does not always provide an "in-process" measurement and, therefore, may not be an accurate determination of heart rate during continuous exertion.

There are 2 sub-types of strapless heart rate monitors. The first type of monitor measures heart rate by detecting electrical impulses. Some wristwatch-type devices have electrodes on the device's underside in direct contact with the skin. These monitors are accurate (often called ECG or EKG accurate) but may be more costly. The second type of monitor measures heart rate by using optical sensors to detect pulses going through small blood vessels near the skin. These monitors based on optical sensors are less accurate than ECG type monitors but may be relatively less expensive.

Optical sensing, related to pulse oximetry, may also be used. The arrangement of heart rate sensor and display may be similar to that described above. The method of measurement is based on a backscattered intensity of light that illuminates the skin's surface and is sensitive to the change of red blood cell density beneath the skin during the pulse cycle. Motion of the sensor may introduce noise that corrupts the signal.

Compensation and removal of noise due to motion of an optical pulse sensor relative to the skin during exercise imposes an additional hardware and signal processing burden on the pulse monitoring device. An apparatus and method of signal processing that compensates and removes noise corrupting the actual pulse, and provides a user friendly apparatus (such as not requiring a chest or ankle sensor, or placement over an artery) would be beneficial and more convenient for physical training.

### SUMMARY

A heart rate monitor is disclosed comprising two main components. A first wristwatch type device measures three categories of sensor signal, digitizes the signals, correlates them to a generated clock signal, encodes them for transmission, and transmits the encoded data to a second device. An exemplary method of transmission may be Bluetooth, although other protocols may be employed, including hard wired signal transmission. The second device may be, for example, a smart phone (e.g., an iPhone™ or equivalent device equipped to transceiver wireless data) or other device, running an application to decode the transmitted data, process the signals to obtain a noise compensated heart rate, store data, and transmit a return signal to the first device on the basis of the processed signals. Additional data may be collected by the first device, such as battery life, pulse signal strength, and the like, which may also be transmitted to the second device. In turn, the second device may return signals to the first device to alert the user with status indicator, such as low battery, pulse rate too high/low, etc. More detailed information may be provided on the display of the second device.

In addition, audio data may be transmitted from the second device to audio earphones either coupled to the first device, or by further receiving a wireless signal such as via Bluetooth™.

In an aspect of the disclosure a signal processing apparatus for determining a heart rate includes a plurality of sensors configured to detect changes in blood properties in a user's skin and heart rate Kalman filter configured to compute a heart rate on the basis of signals obtained from the plurality of sensors.

In an aspect of the disclosure a method of computing a heart rate includes detecting changes in blood properties with a plurality of sensors, and computing with a heart rate Kalman filter the heart rate on the basis of signals obtained from the plurality of sensors.

### BRIEF DESCRIPTION OF THE DRAWINGS

FIG. 1 is a conceptual illustration of a heart rate sensing user system in an aspect of the disclosure.

FIG. 2 is a conceptual illustration of a remote processing system for communicating with and controlling the user system of FIG. 1.

FIG. 3 is a conceptual illustration of a sensing system of the user system of FIG. 1.

FIG. 4 illustrates a conceptual view of the underside of the user system 100.

FIG. 5 illustrates a conceptual view of the front face of the user system 100.

FIG. 6 illustrates a method of operating a heart rate monitor comprising the heart rate sensing user system of FIG. 1 and the remote processing system of FIG. 2.

FIG. 7 is block diagram of the signal processing system of a heart rate sensing system in an aspect of the disclosure.

### DETAILED DESCRIPTION

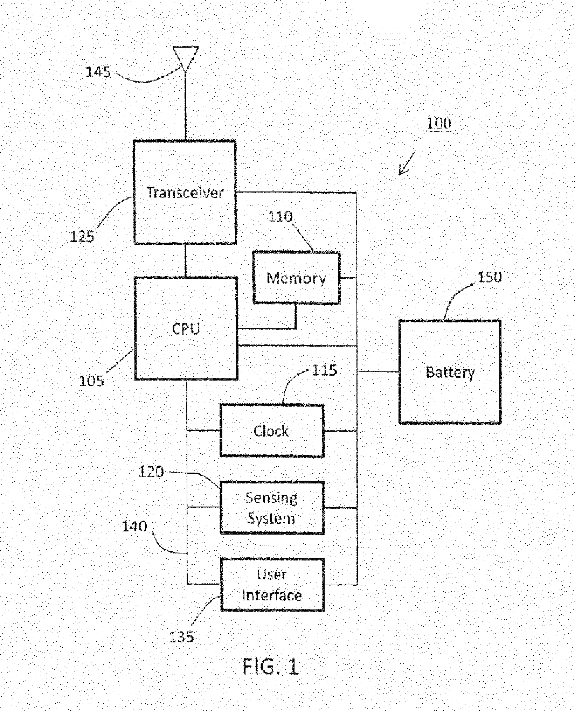

FIG. 1 illustrates a heart rate user system 100. The user system 100 may be worn on a user's wrist, but other locations besides the wrist, such as the ankle, arm or forearm may be used. The user system 100 includes a user processing CPU 105, a user memory 110, a clock signal generator 115, a sensing system 120, a user transceiver 125, and a user interface 135. The CPU 105 may be coupled to the other indicated components, for example, either directly or via a bus 140. A user antenna 145 is coupled to the user transceiver 125. The user antenna 145 may be a wireless connection to a remote processing system (discussed below), or it may be representative of a direct wired connection to the remote processing system.

A battery 150 is coupled to the user processor CPU 105, the user memory 110, the clock signal generator 115, the sensing system 120, the user transceiver 125, and the user interface 135 to power all functions of the indicated elements.

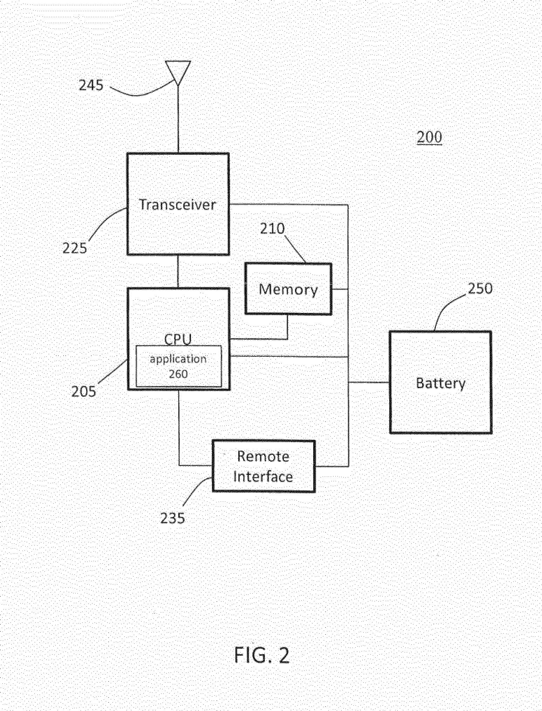

FIG. 2 illustrates a remote processing system 200 for receiving and analyzing signals transmitted from the user system 100. The remote processing system 200 may comprise, for example, a smart phone such as the Apple iPhone™ executing an application program to process the transmitted signals, as will be disclosed in more detail below. The remote processing system 200 may alternatively be a console, such as a dedicate piece of instrumentation for communicating and interacting with the user system 100. For example, the remote console may be used in a hospital, a fitness facility, or the like.

As an exemplary case, the remote processing system 200 may be a smart phone, such as an iPhone™, executing a heart rate monitoring application 260 on a remote CPU 205, where the application 260 may be stored in a remote memory 210 coupled to the remote CPU 205. The remote processing system 200 may also include a remote antenna 245 and a remote transceiver 225. The remote CPU 205 may execute the application commands and process the signals received from the user system 100, and generate output signals to the user system 100 via wireless transmission such as Bluetooth™, or the like, or via hard wire communication, on the basis of the processed received signals.

Alternatively, all processing functions may occur on the user system 100, where all the processing functions of remote processing system 200 may not be occur or be implemented. Further, the distribution of processing functions between the user system 100 and the remote processing system 200 may be split according to system design descisions and still express aspects of the invention.

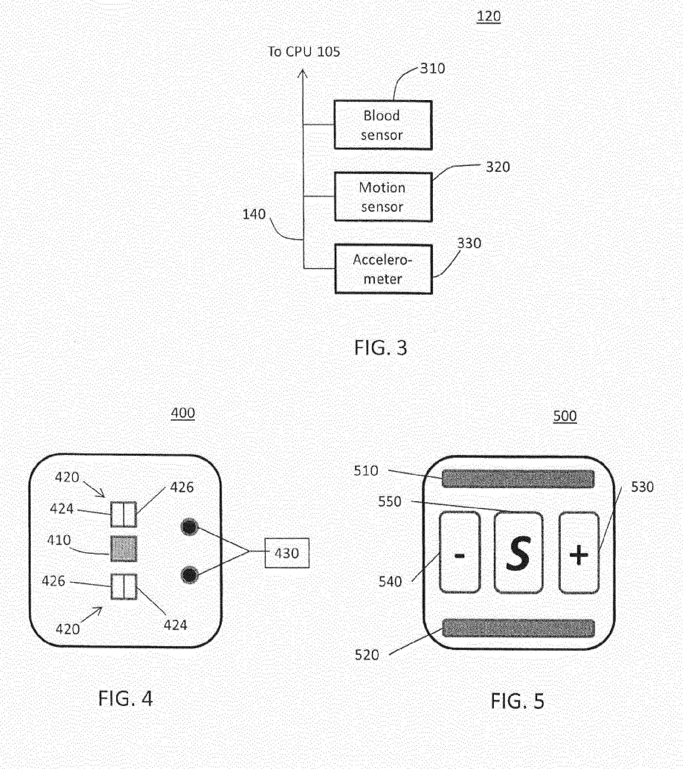

FIG. 3 is a conceptual illustration of the sensing system 120 of the user system 100 of FIG. 1. The sensing system 120 includes a blood concentration sensing system 310, described in more detail below. As the heart pumps blood through the arteries to microscopic blood vessels, the blood concentration varies periodically between a minimum and a maximum concentration, synchronously with a periodic variation in blood pressure. The blood concentration sensing system 310 senses this change in concentration in the blood vessels beneath the skin and transmits the signal level to the CPU 105. The blood concentration sensing system 310 may also sense small motions of the user system 100 with respect to the user's skin, because the blood concentration sensing system 310 may be sensing information from a different area of blood vessels beneath the user's skin if the user system 100 moves relative to the skin. This component of the sensed signal may be regarded as noise, which may contaminate a true determination of the heart rate.

Compensation of this signal is enabled by a sensor that is sensitive to motion, but not to blood concentration. The sensing system 120 further includes a motion sensor 320. The motion sensor functions in a manner analogous to a computer optical mouse, but is relatively insensitive to blood concentration near the surface of the skin. The motion sensor 320 senses changes in the position of the user system 100 with respect to the skin and sends a signal corresponding to that motion to the CPU 105, but contains relatively no substantial signal due to blood concentration. The signal from the motion sensor 320 and the signal from the blood concentration sensing system 310 may be correlated in time with the signal from the clock generator 115 to provide a compensated signal in which the noise contribution due to motion is substantially reduced. The compensated signal may then be analyzed for a more accurate determination of heart rate.

The sensing system further includes an accelerometer 330. The accelerometer 330 may be a chip-set comprising a plurality of sensing elements capable of resolving acceleration along three orthogonal axes. Microelectromechanical system (MEMS) sensors, capacitive sensors, and the like, are well known in the art of acceleration sensing. The accelerometer 330 may provide information about the motion of the user system 100 with respect to the user's heart. For example, if the user system 100 is worn on the wrist, and the nature of the exercise requires the wrists and hands to rise above the heart, the consequent elevation may cause a drop in the minimum and maximum (min/max) of the blood pressure at the point of sensing relative to that which may be measured when the user system 100 is as the same level or lower than the heart. This information may be used to qualify or disqualify the blood concentration measurements if the measured min/max values fall outside an acceptable range for determining the heart rate.

Some judgment may be used in making most effective use of the accelerometer 330. For example, if the exercise comprises bench presses, where the user's arms and hands are constantly being raised above the chest, placement of the user system at a relatively motion neutral location, such as an ankle or upper calf. The signal measured by the accelerometer 330 will not then indicate a shifting "baseline" for the effect of blood pressure on blood concentration measurements due to altitude change relative to the heart, and more data will qualify.

FIG. 4 illustrates a conceptual underside view 400 of the user system 100, showing elements of the blood concentration sensing system 310 and the motion sensor 320. In the illustration shown in FIG. 4, a photodetector 410 is positioned between two sets 420 of light emitting diodes (LEDs), although other light sources may be contemplated within the scope of the invention. Only one set 420 of LEDs is required, as a minimum, as discussed below, but a plurality of such LEDs can improve the sensitivity and performance of the user system 100. The photodetector 410 and the LED set 420 are positioned in close proximity, e.g., adjacent, to the user's skin and close to each other. Light emanating from an LED in the set 420 will penetrate a limited skin depth and a portion of the penetrating light will backscatter and be detected by the photodetector 310. As will be described below, the photodetector 410 has a spectral sensitivity that spans at least from green to red, or at least spanning the spectral bandwidths of the two LEDs.

For operation of the blood concentration sensing system 310, the LED set 420 includes a green LED 424. Green light is preferentially absorbed by red blood cells in the skin. Therefore, a systolic increase in blood pressure and vascular blood concentration during the course of a pulse may result in a decreased backscattered green light intensity. During the diastolic interval, blood concentration is lower, leading to an increased backscattered green light. The sensed signal level provided by the photodetector 410, when synchronized with the clock signal generator 115, may be analyzed under the control of a computer program stored in the user memory 110 and executable on the CPU 105 to determine a periodicity of the min/max signals, and thus determine a heart rate. Alternatively, signal may be exported to the remote user system 200, where the remote processing system 200 performs substantially the same functions.

During exercise, a degree of motion of the user system 100 along the skin may occur. Because this changes the detailed microvascular network illuminated by the green LED 424, a motion signal, which may be regarded as noise, may be included in the backscattered green light. Therefore, a motion sensor independent of blood concentration is beneficial.

For operation of the motion sensor 320, the LED set 420 includes a red LED 426. Red light backscattered from vascular tissue in the skin is not substantially affected changes in blood concentration, and is not substantially sensitive to the pulsing of blood near the skin surface. However, the red LED 426 and photodetector 410 may function in a manner similar to an optical mouse, which is sensitive to motion relative to a surface, which in the present case happens to be the user's skin. The red LED 426 is used to sense small motion of the sensor with respect to the microvascular structure just beneath the skin. In an embodiment of the implementation of the motion sensor 320, the photodetector 410 may be a special purpose image processing chip that measures pixel-to-pixel changes in light intensity to compute motion of the user system relative to the user's skin. Such motion is to be expected in the course of exercise. This may result in a variation in signal levels having a temporal spectrum consistent with the periodicity of physical motion and which corrupts the primary heart rate signal of interest.

Operation of both the blood concentration sensing system 310 and the motion sensor 320 with a common photodetector 410 is achieved by alternately firing the green LED 424 and the red LED 426 under control of the CPU 105, synchronized with the clock signal generator 115. Thus, the photodetector 410 must have sensitivity to spectral bands including both LED colors. The clock signal rate may be high enough, e.g., typically a kilohertz or more, that the two signals - for blood concentration and motion - may appear to be quasi-continuous, with enough granularity to extract sufficient detail from each - i.e., blood concentration and motion from the green LED 424 and motion only from the red LED 426. Additionally, there may be included a time interval between red and green pulses when neither LED is fired, enabling the photodetector to acquire an ambient "background" signal that may include, for example, fluorescent lighting, wireless or circuitry generated signals that constitute noise added to the system user 100 signal in addition to the signal detected by the concentration sensing system 310, motion sensor 320 and accelerometer 330.

One of the functions of the user CPU 105 may further include reading the battery level to the CPU 205 of the remote processing system 200 as transmitted, for example, via Bluetooth™, and returning a command to the user system 100 to display an indication that the battery level is normal or low.

Another function of the remote CPU 205 may be to determine, on the basis of the received sensor signals, whether the pulse signal peak values are too large (causing saturation) or two weak (causing poor signal-to-noise ratio (SNR)). If the detected pulse is two weak, the remote CPU 205 may provide feedback to the user CPU 105 instructing it increase the intensity of the LEDs by increasing the pulse peak power or pulse width, or reducing the intensity of the LEDs by reducing the pulse peak power or pulse width if the signal is saturating. This is especially valuable because normative values of blood pressure may differ for different people, e.g., different skin pigment and light absorption properties, and may also change significantly as the course of a variable exercise regimen progresses through different levels of activity. For example, when the user is engaged in a sports activity, blood pressure and blood concentration is usually higher, so less light may be required to pick up a signal. Therefore, the pulse driven fluctuation of the green LED light is affected by blood pressure, and the current to the green LED may be controlled to conserve power and prevent signal saturation.

Alternatively, as mentioned above, this function may be performed locally on the user system 100. In this alternative configuration, the user CPU 105 may be to determine, on the basis of the sensor signals output from the photodetector 410, or the power applied to the LEDs, whether the pulse signal peak values are too large (causing saturation) or two weak (causing poor signal-to-noise ratio (SNR)). If the detected pulse is two weak, the user CPU 205 may increase the pulse peak power or pulse width, or reduce the pulse peak power or pulse width if the signal is saturating.

As described earlier, the operation of the blood concentration sensing system 310 and the motion sensor 320 with a common photodetector 410 may be achieved by alternately firing the green LED 424 and the red LED 426 under control of the CPU 105. In an alternative configuration of the sensing system, the firing sequence may include a blanking period after the green and red LEDs are fired. In this configuration, the user CPU 105 will cause, by way of example, the green LED 424 to fire, followed by the red LED 426, and then followed by a blanking period before the sequence repeats. The remote CPU 205 may then determine on the basis of the received sensor signal for the blanking period the effect that sunlight, fluorescent light or stray electronic emissions are having on the measurements. The remote CPU 205 may then compensate the received sensor signals for the green and red LEDs when computing the heart rate of the user, or provide this information back to the user CPU 105 in the form of feedback for adjusting the intensity of the green and red LEDS.

The user system 100 as shown in the underside view 400, may also include recharging ports 430 for recharging the user battery 150.

Using the remote interface 235 of the remote processing system 200, an exercise schedule may be created. The remote interface 235 may be, for example, a touch screen, such as found on an APPLE iphone™, a smart phone keyboard and screen, and a screen, keyboard and mouse of a computer console. A maximum estimated heart rate may be determined based on various factors, including the user's age. A maximum estimated heart rate may correspond to an extreme level of performance, and different levels of performance may correspond to different ranges spanning from the maximum estimated heart rate down to a range corresponding to a resting state, so that a range of heart rates may be established for each range of exercise performance. Typical ranges of performance may correspond to resting, moderate exercise (e.g., walking), up to an extreme range corresponding to a maximum recommended level of activity, keeping in mind that such levels are only guidelines, and subject to appropriate modification. Having chosen a level of exercise, the remote processing system 200 CPU 205 may communicate via the transceivers 145 and 245 to the user system CPU 105 to signal when the received sensor signals indicate the heart rate is below, within, or above the selected exercise performance range. In this manner, the user may control and monitor his/her level of activity.

Referring now to FIG. 5, illustrating a conceptual view of the front face 500 of the user system 100, the user CPU 105, on the basis of performance range information received from the remote system 200, may control display features on the front face 500, away from the user's skin, which is thus accessible to the user. For example, in one embodiment, a red light indicator 510 on the display face may indicate that the heart rate is above a prescribed range for a selected exercise performance, and the user should exercise more slowly. Conversely, a green light indicator 520 may indicate that the performance level is below the prescribed range, and the user should exercise harder. At an appropriate level of exercise, neither light may be on, indicating an appropriate level of exercise is obtained. Other combinations of light indicators and colors may me contemplated within the scope of the invention.

Additional functionality may be included in the user system 100 in coordination with functionality available in the remote processing system 200. For example, the remote processing system 200 may also serve as an audio player (MP3, iPod™, etc.) storing a number of music tracks, or accessing a number of radio stations, made available by an appropriate entertainment software application running on the remote processing system 200. Referring to FIG. 5, a set of buttons ("+" = volume up/track forward 530, "-" = volume down/track backward 540, and "select" S 550) on the user system 100 front face 500 enable the user to select an audio file or channel and volume. The select button S 550 may provide entertainment selection functions, such as pause, play, etc.

Additionally, the select button S 550 may serve as an emergency alert button. For example, repeated or continuously press S 550 may initiate a signal from the user system 100 to the remote processing system 200 to activate an alarm, such as an emergency alert phone message (911, private physician, or the like). If the remote processing system 200 is also equipped with GPS, the emergency alert message may also contain the location of the user, and vital statistics, such as the heart rate and/or high or low blood concentration level, which may indicate a high or low blood pressure, together with the identity of the user.

The remote system 200 may be carried by the user, for example, on a wrist, arm or waist strap, with viewing access easily available. The remote system 200 may therefore provide on its display (not shown) more detailed information, such as heart rate, calories burned, distance run, and the like, as determined by the application.

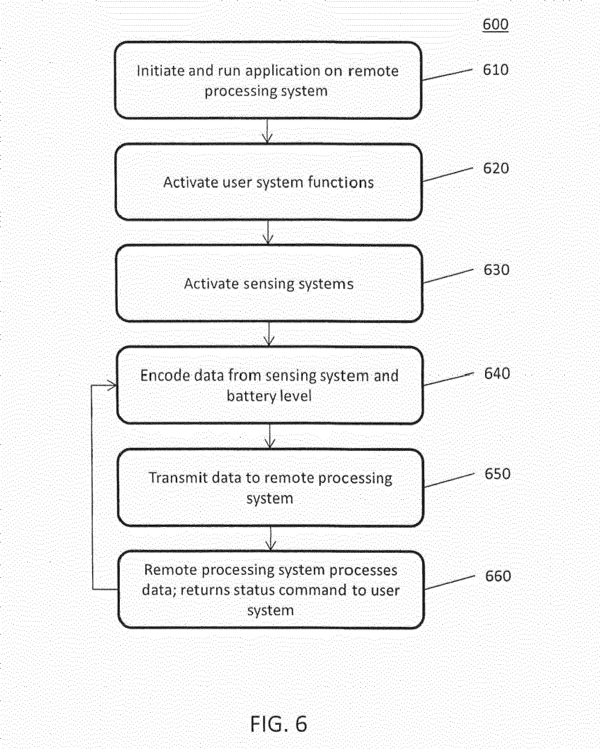

FIG. 6 illustrates a method 600 of operating the heart rate monitor comprising the user system 100 and the remote processing system 200. In block 610, the user initiates and runs the heart rate monitoring application 260 on the remote processing system 200. In block 620 the remote processing system 200 communicates with and activates the user system 100 heart monitor functions stored in the user memory 110 executable on the user CPU 105. The user system CPU 105 turns on operation routines controlling the sensing system 120 comprising the green LED 424, the red LED 426 and photodetector 410 and also the accelerometer operation routines in block 630. The routines control the operation of the LEDs, i.e., the repetition rate, alternating timing of the green and red LEDs, pulse widths of the LED output, and photodetector circuitry. The routines may also control the operation of the accelerometer 330 and associated circuitry. In block 640 the CPU 105 converts the analog signal from the photodetector , the accelerometer and the battery voltage to a digital signal that is then encoded for transmission as a data packet. In block 650, a signal is transmitted by the user system 100 CPU via the transceivers 225, 245, such as a Bluetooth™, and antennas 245, 345 to the remote processing system 200 including the blood concentration data, motion data accelerometer data, battery voltage, and clock signal. Alternatively, transmission may be via a hard wire link. In block 660, the remote processing system 200 CPU 205 processes the received data and may transmit various commands back to the user system 100 CPU 105. Among these include commands to turn on red or green LEDs on the front face of the user system to indicate to the user to exercise faster (green LED), exercise slower (red LED), and maintain the same level of exercise (no front LED lit).

The method functions continuously by returning, for example, to block 640, to obtain and encode the next packet of data.

The battery level may be indicated during charging. For example, when the user system is being charged through the charging ports 430, the green LED 510 may blink intermittently once for 25% charged, twice for 50% charged, three times for 75% charged, and steady on for 100% charged, or the like.

All operation conditions and exercise parameters may be visually presented on the user interface of the remote processing device 200, e.g., the touch screen of an iPhone™ or computer screen.

The remote processing device 200 display (not shown) may show a variety of data. Exemplary information that may be displayed include a numeric value of the measured (corrected) heart rate, a workout time indicator, a calorie counter, a level of performance indicator, exercise, pause and stop soft keys, and a music function soft key, all accessible using the multifunction key. Other functions may be contemplated as well.

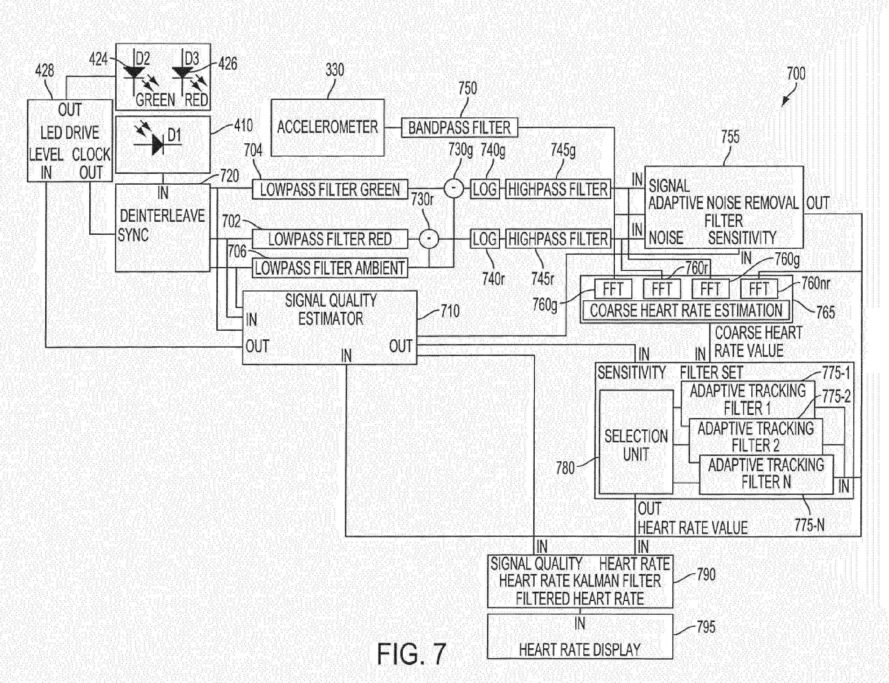

FIG. 7 illustrates a conceptual diagram of a system 700 for controlling sensors, processing data and detecting heart rate accurately. As indicated above, the system 700 includes green LEDs 424, red LEDs 426, photodector 410, and accelerometer 330. The LEDs are driven by an LED driver 428. The LED drive levels and timing are provided by a Signal Quality Estimation block 710, described in more detail below. Signals received by the detector 410 (including red pulses, green pulses and "off" pulses) are separated in time according to a clock output of the LED driver 428, by a signal de-interleaver synched to the LED driver clock, which outputs in separate channels the detected red, green and ambient signals. The detector may be coupled to the signal de-interleaver via a fixed analog amplifier and a voltage level adjustment circuit to provide a desired level of detected red and green signal in a satisfactory range for signal processing. However, other means of signal amplification and adjustment may be used and are considered within the scope of the disclosure.

Each of the red green and ambient signals are separately passed through corresponding lowpass filters to remove high frequency noise not associated with blood flow rates consistent with the possible ranges of physical activity such as, for example, ac ambient light. The filtered ambient signal is subtracted from the red and green signals to remove ambient artifacts.

The separate red green and ambient signals (before lowpass filtering) are also input to a Signal Quality Estimator, which determines if the red and green detected signals are too weak or saturating. Based on the results, the Signal Quality Estimator provides level control instructions to the LED Driver to adjust the output of the LEDs accordingly.

Returning to the lowpass filtering section, the ambient adjusted red and green lowpass filtered signals are each separately converted to a logarithm scale output and passed through a highpass filter. Conversion of the light signals to log scale enables signal normalization to maintain the heart rate AC component of amplitude in the same range. The filtering step removes, at least, any DC offsets and drift caused by, for example, skin or sensor-to-skin changes in the signal and any low frequency noise not associated with the frequencies related to heart rate and rhythmic physical motion.

The accelerometer outputs a signal in response to acceleration due to physical motion. This signal may be a source of noise that is imposed on the red and green signals. The accelerometer signal is passed through a bandpass filter to remove and DC offsets and high frequency noise similar to that discussed above for the red and green signals.

The highpass filtered log scale red and green signals are input to an adaptive noise removal filter. The noise signal is supplied by the bandpass filtered accelerometer signal. The adaptive noise removal filter digitizes the input filtered red, green, and accelerometer signals and self-adjusts its transfer function according to an optimization algorithm driven by an error signal. The error signal may be supplied by the signal quality estimator. The output is an adaptively filtered noise removal green light signal, the color sensitive to changes in blood concentration.

The filtered red, green, and accelerometer signals (prior to entering the adaptive noise removal filter), and the adaptively filtered noise removal signal are each input into separate Fast Fourier Transform (FFT) processor channels in a coarse heart rate estimator. An output of a coarse heart rate value is an estimate of the actual heart rate done by selecting the frequency out of the fit spectrum that is most probably the heart rate, taking into account the amounts of noise on the other channels.

The coarse heart rate value, in the form of the four FFT spectra is input to a filter set of adaptive tracking filters that self-adjust respective transfer functions according to an optimization algorithm driven by the error signal supplied by the signal quality estimator. The adaptive tracking filters actively adjust the weight of how much of the accelerometer FFT spectrum to subtract from the red and green spectra on the basis of the filtered red FFT, filtered green FFT, filtered accelerometer FFT and the adaptively filtered noise removal signal FFT. The output of each of the adaptive tracking filter is heart rate value determined on the basis of the optimization algorithm of each of the adaptive tracking filters. A selection unit selects which adaptive tracking filter output to provide as the heart rate value on the basis of the error signal from the signal quality estimator and the signal from the adaptive noise removal filter.

The heart rate value output from the selection unit of the filter set and the error signal from the signal quality estimator may be input to a Kalman filtering unit to provide a filtered hear rate. The filtered heart rate may then be output, for example, to a display for a user to read.

The distribution of processing functions between a user system and a remote system may vary according to design and functional requirements. For example, the user system may include only the LEDs, LED driver, de-interleaver and accelerometer, where the acquired analog signals are digitized and transmitted to a remote system for processing and determination of the filtered heart rate. The filtered heart rate may then be transmitted back to the user system for display to the user. At the other extreme, all processing functions may be executed entirely on the user system, and the filtered heart rate displayed to the user. Data may be temporarily stored on the user system and then transmitted (periodically or on demand) for download to a remote system for archiving or further processing. Alternatively, an intermediate level of signal processing may be performed on the user system and the balance performed on the remote system.

It is to be understood that such a system may be applied beyond the example given of a heart rate monitor, such as, for example, gaming, social networking, emergency alarming, etc.
It is to be understood that the specific order or hierarchy of steps in the methods disclosed is an illustration of exemplary processes. Based upon design preferences, it is understood that the specific order or hierarchy of steps in the methods may be rearranged. The accompanying method claims present elements of the various steps in a sample order, and are not meant to be limited to the specific order or hierarchy presented unless specifically recited therein.

The claims are not intended to be limited to the various aspects of this disclosure, but are to be accorded the full scope consistent with the language of the claims. All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112, sixth paragraph, unless the element is expressly recited using the phrase "means for" or, in the case of a method claim, the element is recited using the phrase "step for."

Other non-limiting aspects of the invention are as follows:
A. A method of computing a heart rate, comprising:
   detecting changes in blood properties with a plurality of sensors; and
   computing with a heart rate Kalman filter a heart rate on the basis of signals obtained from the plurality of sensors.
B. The method of clause A, wherein the plurality of sensors comprise:
   an optical sensor comprising a plurality of light emitting devices and one or more photodetectors; and
   an accelerometer, the method further comprising:
      detecting with the photodetectors optical signals from the light emitting devices and ambient light; and
      detecting motion adding a noise signal to the optical signals.
C. The method of any of clause A or B, further comprising controlling with a driver a periodic emission level and timing of light output from the light emitting devices and a period of off state of the light emitting devices.
D. The method of any of clauses A to C, further comprising determining with a signal quality estimator a drive level of the light emitting devices on the basis of the detected optical signals.
E. The method of any of clauses A to D, wherein a one or more of the plurality of filters is configured to filter noise outside specified frequency bands from the optical signals, comprising subtracting filtered noise from an ambient signal when the light emitting devices are off from the optical signals when the light emitting devices are on.
F. The method of any of clauses A to E, further comprising removing, with an adaptive noise removal filter, noise on the basis of the accelerometer noise signal from the noise filtered optical signals and provide a noise removal filtered signal.
G. The method of any of clauses A to F, further comprising:
   providing, with a spectrum analyzer, spectra of the optical signals, accelerometer noise signal, and noise removal filtered signal; and
   determining a coarse rate heart estimation on the basis of the spectra.
H. The method of any of clauses A to G, further comprising determining, with a plurality of adaptive tracking filters, a heart rate value on the basis of the coarse heart rate estimation, the error signal from the signal quality estimator and the noise removal filtered signal.
I. The method of any of clauses A to H, further comprising computing a heart rate with the heart rate Kalman filter to compute the heart rate on the basis of an error signal from the signal quality estimator and the heart rate value from the plurality of adaptive tracking filters.

## Claims

1. A signal processing system comprising:
a sensor means for generating a plurality of sensor signals;
a means for Kalman filtering configured to compute a heart rate on the basis of the sensor signals.

2. A signal processing system to determine a heart rate, comprising a signal processing system as claimed in claim 1, wherein:
the sensor means comprises a plurality of sensors configured to detect changes in blood properties in a user's skin; and
the means for Kalman filtering is a heart rate Kalman filter configured to compute a heart rate on the basis of signals obtained from the plurality of sensors.

3. The system of claim 1 or 2, wherein the sensor means comprises:
an optical sensing means comprising a plurality of light emitting devices and one or more photodetectors, wherein the photodetector is configured to detect optical signals from the light emitting devices and ambient light; and
an acceleration sensing means configured to detect motion adding a noise signal to the optical signals.

4. The system of claim 3, further comprising means for removing an optical signal noise due at least to the ambient light and the motion noise signal to compute a heart rate.

5. The system of claim 3 or 4, further comprising a driver to control a periodic emission level and timing of light output from the light emitting devices and a period of off state of the light emitting devices.

6. The system of claim 3, 4 or 5, further comprising a signal quality estimator to determine a drive level of the light emitting devices on the basis of the detected optical signals.

7. The system of any of claims 3 to 6, wherein a one or more of a plurality of filters is configured to filter noise outside specified frequency bands from the optical signals, and wherein filtered noise from an ambient signal when the light emitting devices are off is subtracted from the optical signals when the light emitting devices are on.

8. The system of claim 7, wherein the acceleration sensing means is an accelerometer and further comprising an adaptive noise removal filter configured to remove from the noise filtered optical signals noise on the basis of the accelerometer noise signal and provide a noise removal filtered signal.

9. The system of claim 8, further comprising a spectrum analyzer to provide spectra of the optical signals, accelerometer noise signal, and noise removal filtered signal, wherein the spectra provide a coarse rate heart estimation.

10. The system of claim 9, further comprising a plurality of adaptive tracking filters to determine a heart rate value on the basis of the coarse heart rate estimation, the error signal from the signal quality estimator and the noise removal filtered signal.

11. The system of claim 10, wherein the heart rate Kalman filter is configured to compute a heart rate on the basis of an error signal from the signal quality estimator and the heart rate value from the plurality of adaptive tracking filters.

12. A method of computing a heart rate, comprising:
detecting changes in blood properties with a plurality of sensors; and
computing with a heart rate Kalman filter a heart rate on the basis of signals obtained from the plurality of sensors.

13. The method of claim 12, wherein the plurality of sensors comprise:
an optical sensor comprising a plurality of light emitting devices and one or more photodetectors; and
an accelerometer, the method further comprising:
detecting with the photodetectors optical signals from the light emitting devices and ambient light; and
detecting motion adding a noise signal to the optical signals.

14. The method of claim 13, further comprising controlling with a driver a periodic emission level and timing of light output from the light emitting devices and a period of off state of the light emitting devices.

15. The method of claim 13 or 14, further comprising determining with a signal quality estimator a drive level of the light emitting devices on the basis of the detected optical signals.
